(19) Europäisches Patentamt
European Patent Office
Office européen des brevets

(11) **EP 3 600 482 B1**

(12) **EUROPÄISCHE PATENTSCHRIFT**

(45) Veröffentlichungstag und Bekanntmachung des Hinweises auf die Patenterteilung:
**07.07.2021   Patentblatt 2021/27**

(21) Anmeldenummer: **18713841.7**

(22) Anmeldetag: **20.03.2018**

(51) Int Cl.:
*A61M 1/14* (2006.01)          *A61M 1/36* (2006.01)
*H01L 41/113* (2006.01)

(86) Internationale Anmeldenummer:
**PCT/EP2018/057057**

(87) Internationale Veröffentlichungsnummer:
**WO 2018/172372 (27.09.2018 Gazette 2018/39)**

(54) **EINRICHTUNG MIT EINRICHTUNG ZUM ERMITTELN EINER, INSBESONDERE GEOMETRISCHEN, EIGENSCHAFT DER EINRICHTUNG**

APPARATUS COMPRISING DEVICE FOR DETERMINING AN ESPECIALLY GEOMETRICAL PROPERTY OF THE APPARATUS

ÉQUIPEMENT DOTÉ D'UN DISPOSITIF DE DÉTERMINATION D'UNE CARACTÉRISTIQUE, EN PARTICULIER GÉOMÉTRIQUE, DE L'ÉQUIPEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.03.2017   DE 102017106404**

(43) Veröffentlichungstag der Anmeldung:
**05.02.2020   Patentblatt 2020/06**

(73) Patentinhaber: **Fresenius Medical Care Deutschland GmbH**
**61352 Bad Homburg (DE)**

(72) Erfinder:
• **HEIDE, Alexander**
**65817 Eppstein (DE)**
• **NIKOLIC, Dejan**
**65812 Bad Soden (DE)**

(74) Vertreter: **Bobbert & Partner Patentanwälte PartmbB**
**Postfach 1252**
**85422 Erding (DE)**

(56) Entgegenhaltungen:
DE-A1- 19 605 652          US-A- 4 397 194
US-A1- 2003 042 181          US-A1- 2011 214 504
US-A1- 2014 263 064

EP 3 600 482 B1

**Beschreibung**

Die vorliegende Erfindung betrifft eine Einrichtung, insbesondere eine medizinische Einrichtung, gemäß dem Oberbegriff des Anspruchs 1. Sie betrifft ferner ein Verfahren gemäß dem Oberbegriff des Anspruchs 12 und eine Auswerteeinheit, eine Behandlungsvorrichtung gemäß dem Oberbegriff des Anspruchs 14 sowie ein Set gemäß Anspruch 15.

Bekannte Blutbehandlungsvorrichtungen werden zur Blutbehandlung mit wenigstens einer medizinischen Einrichtung verbunden, beispielsweise in Gestalt einer Blutkassette, in welcher Blut behandelt oder vorübergehend gespeichert wird. Als Blutkassetten ausgestaltete medizinische Einrichtungen sind aus der DE 10 2009 018 664 A1 bekannt.

Aus der US 2003/0042181 A1 sind eine Messvorrichtung und ein Messverfahren zur Bestimmung von Parametern von medizinischen Fluids bekannt.

In der US 2014/0263064 A1 sind medizinische Fluidsensoren sowie verwandte Systeme und Methoden offenbart.

Aus der US 2011/0214504 A1 sind Einwegdrucksensorsysteme und Packarten hierzu bekannt.

Aufgabe der vorliegenden Erfindung ist, eine weitere Einrichtung, insbesondere medizinische Einrichtung, anzugeben. Ferner soll ein Verfahren für die Verwendung einer solchen Einrichtung vorgeschlagen werden, eine Auswerteeinheit und eine Behandlungsvorrichtung.

Diese Aufgabe kann durch eine Einrichtung oder eine medizinische Einrichtung mit jeweils den Merkmalen des Anspruchs 1 und durch ein Verfahren mit den Merkmalen des Anspruchs 12 gelöst werden, ferner durch eine Behandlungsvorrichtung mit den Merkmalen des Anspruchs 14 sowie durch ein Set mit den Merkmalen des Anspruchs 15.

Erfindungsgemäß wird somit eine Einrichtung, insbesondere medizinische Einrichtung, mit wenigstens einem Hartteil mit vollständigen oder unvollständigen Fluidbahnen zum Führen eines Fluids, insbesondere eines medizinischen Fluids, insbesondere Blut, im Hartteil oder durch das Hartteil hindurch, angegeben.

Die medizinische Einrichtung weist ferner wenigstens einen Wandler auf. Der Wandler ist angeordnet zum Messen einer Eigenschaft des medizinischen Fluids oder eines anderen Fluids, während dieses in einer der Fluidbahnen vorliegt. Alternativ oder ergänzend ist der Wandler angeordnet zum Messen einer Eigenschaft der medizinischen Einrichtung oder einer Einwirkung wie einen von außen oder innen aufgebrachten Druck auf letztere.

Die medizinische Einrichtung weist ferner eine Einrichtung zum Ermitteln einer Eigenschaft, insbesondere einer geometrischen Eigenschaft, der medizinischen Einrichtung, insbesondere einer Fluidbahn hiervon, auf. Sie kann optional als Sensor, etwa zum Bestimmen einer Geometrie, einer Passgenauigkeit, eines Volumens, einer Erstreckung usw. ausgestaltet sein. Sie kann optional mechanisch (Druck), optisch, mittels Schallmessung oder Ultraschallmessung und/oder auf anderem Wege die Eigenschaft bestimmen.

Das erfindungsgemäße Verfahren dient dem Messen oder Bestimmen einer Eigenschaft eines in einer medizinischen Einrichtung vorliegenden Fluids, z. B. mittels eines Wandlers einer medizinischen Einrichtung. Alternativ oder ergänzend dient das Verfahren dem Ermitteln oder Ausgeben eines Werts einer Eigenschaft eines Fluids, etwa eines medizinischen Fluids. Es dient vorzugsweise dem Korrigieren einer unter Einsatz des Wandlers und eines von diesem abgegebenen Signals ermittelten Eigenschaft des Fluids oder dem Korrigieren des Signals.

Das Verfahren umfasst ein Bereitstellen einer erfindungsgemäßen medizinischen Einrichtung.

Es umfasst weiter das Ausgeben eines Signalwerts mittels des Wandlers der medizinischen Einrichtung in Relation zu einer Eigenschaft des Fluids, während dieses in einer der Fluidbahnen vorliegt.

Das Verfahren umfasst ferner das Bereitstellen des Ergebnisses einer Referenzmessung für die Eigenschaft, insbesondere eine geometrische Eigenschaft. Dieses Ergebnis wird hier als "erster Wert" bezeichnet. Die Referenzmessung, die zu diesem Ergebnis geführt hat, kann vorab, also vor Beginn des erfindungsgemäßen Verfahrens erfolgt sein. Sie kann werkseitig, vor Auslieferung an den Verbraucher, usw. erfolgt sein, ohne hierauf beschränkt zu sein.

Zum Verfahren zählt ferner das Empfangen eines Signals von der Einrichtung zum Ermitteln der, insbesondere geometrischen, Eigenschaft der medizinischen Einrichtung, wobei das Signal die, insbesondere geometrische, Eigenschaft betrifft. Die Höhe des Signals oder ihr Wert wird hierin als "zweiter Wert" bezeichnet.

Schließlich umfasst das Verfahren auch das Ausgeben eines Werts der Eigenschaft des Fluids, insbesondere des medizinischen Fluids, unter Verwenden einer Relation zwischen dem ersten Wert und dem zweiten Wert. Alternativ oder ergänzend umfasst das Verfahren das Korrigieren des Signalwerts unter Verwenden der Relation zwischen dem ersten Wert und dem zweiten Wert.

Die offenbarte Auswerteeinheit oder Auswerteeinrichtung, ist programmiert oder konfiguriert zum Ausführen des erfindungsgemäßen Verfahrens, optional im Zusammenwirken mit einer erfindungsgemäßen Behandlungsvorrichtung und einer erfindungsgemäßen medizinischen Einrichtung.

Die erfindungsgemäße Behandlungsvorrichtung weist eine Einrichtung auf zum Aufnehmen oder Ankoppeln der erfindungsgemäßen medizinischen Einrichtung an die Behandlungsvorrichtung. Letztere Einrichtung kann eine Aktor-Sensor-Platte sein oder aufweisen.

Die Behandlungsvorrichtung weist ferner eine Einrichtung zum Bereitstellen des Ergebnisses einer Referenzmessung für die, insbesondere geometrische, Ei-

Die Behandlungsvorrichtung weist eine Einrichtung auf zum Empfangen eines Signals von der Einrichtung zum Ermitteln einer, insbesondere geometrischen, Eigenschaft der medizinischen Einrichtung. Diese Empfangs-Einrichtung empfängt ein Signal, das die, insbesondere geometrische, Eigenschaft betrifft. Das Signal wird hierin als der "zweite Wert" verstanden.

Die Behandlungsvorrichtung weist schließlich eine Einrichtung zum Ausgeben eines Werts der Eigenschaft des, insbesondere medizinischen, Fluids, unter Verwenden einer Relation zwischen dem ersten Wert und dem zweiten Wert, auf. Alternativ oder ergänzend weist die Behandlungsvorrichtung eine Einrichtung auf zum Korrigieren des Signalwerts unter Verwenden der Relation zwischen dem ersten Wert und dem zweiten Wert.

Alternativ oder ergänzend zu den vorstehend genannten Einrichtungen weist die Behandlungsvorrichtung eine Auswerteeinheit auf.

Bei allen Ausführungen hierin ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Erfindungsgemäße Ausführungsformen können eines oder mehrere der vorstehend oder im Folgenden genannten Merkmale aufweisen. Dabei können die hierin genannten Merkmale in beliebigen Kombinationen Gegenstand von erfindungsgemäßen Ausführungsformen sein, sofern der Fachmann eine konkrete Kombination nicht als technisch unmöglich erkennt. Erfindungsgemäße Ausführungsformen sind ferner Gegenstand der Unteransprüche.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

Wenn hierin von einer Einrichtung die Rede ist, die zum Ausführen eines Verfahrensschritts dient, so kann die betreffende Einrichtung zum Ausführen dieses Schritts jeweils konfiguriert, programmiert, vorgesehen und/oder ausgestaltet sein.

In einigen beispielhaften erfindungsgemäßen Ausführungsformen sind unter dem Hartteil (hierin auch als Hartkörper oder als Substrat bezeichnet) der in der Regel in einem Spritzgussverfahren hergestellte und daher hierin als "hart" bezeichnete Korpus der medizinischen Einrichtung, welcher mit einer vergleichsweise "weichen" Folie bedeckt sein kann, und/oder seine Anhänge wie Schläuche usw., zu verstehen. Das Hartteil kann aus PP (Polypropylen), PE (Polyethylen), PA, ABS, PMMA, PC, PVC oder anderen dem Fachmann hinlänglich bekannten Polymeren oder anderen Materialien gefertigt sein. Es kann aus Isolatormaterialien, wie insbesondere z. B. Keramik, gefertigt sein.

Im Rahmen der vorliegenden Beschreibung werden die Begriffe "Sensor" und "Sensoranordnung" gleichbedeutend für ein System mit oder aus einem Wandler, Signalübertragungskomponenten und Auswerteeinheit verwendet. Ein Wandler ist dabei vorzugsweise als ein einrichtungsseitig vorgesehener Abschnitt des Sensors zu verstehen, der mit dem Messmedium in Kontakt steht. Er wandelt das Ergebnis seiner Messung in geeignete Signale, z. B. Strom- oder Spannungssignale. Die ebenfalls zur Sensoranordnung zählenden Signalübertragungskomponenten leiten das Ergebnis seiner Messung an die Auswerteeinheit weiter, die im Stand der Technik üblicherweise maschinenseitig vorgesehen ist.

In einigen beispielhaften erfindungsgemäßen Ausführungsformen ist die Eigenschaft der medizinischen Einrichtung eine geometrische Eigenschaft. Die Eigenschaft der medizinischen Einrichtung wird im Folgenden auch kurz als "Eigenschaft" bezeichnet. Sie kann eine geometrische Eigenschaft sein, ist hierauf aber nicht beschränkt.

Ferner können unter "Eigenschaft" auch die räumliche Ausdehnung, die Größe oder die Beschaffenheit der medizinischen Einrichtung verstanden werden. Ferner kann ebenso die Materialdichte und, z. B. daraus abgeleitet, auch die Güte der Sterilisation als Eigenschaft bezeichnet werden. Ferner wird/werden unter Eigenschaft optional auch die Materialfarbe oder die chemischen Eigenschaften wie die Zusammensetzung oder enthaltene Partikel verstanden. Ferner sind optional auch die Material- und/oder die Wandstärke als Eigenschaft der medizinischen Einrichtung zu verstehen.

In einigen beispielhaften erfindungsgemäßen Ausführungsformen ist die Einrichtung zum Ermitteln einer Eigenschaft der medizinischen Einrichtung eine Einrichtung, konfiguriert und/oder programmiert und/oder ausgestaltet zum Ermitteln einer, insbesondere geometrischen, Eigenschaft der medizinischen Einrichtung.

In einigen beispielhaften erfindungsgemäßen Ausführungsformen ist die Einrichtung zum Ermitteln einer Eigenschaft der medizinischen Einrichtung, insbesondere einer Fluidbahn, eine einteilige oder mehrteilige Komponente, welche die Eigenschaft bestimmen oder messen kann. In solchen Ausführungsformen kann die Einrichtung jeweils ein Sensor oder ein Wandler sein, der Ergebnisse einer von ihm oder mit ihm durchgeführten Messung abgibt. In solchen Ausführungsformen kann die Einrichtung wenigstens je eine Sensorik, eine Schaltung, einen Leiter oder dergleichen aufweisen. In solchen Ausführungsformen könnte man die Einrichtung als eine "aktive" Einrichtung bezeichnen.

In einigen beispielhaften erfindungsgemäßen Ausführungsformen ist die Einrichtung zum Ermitteln einer Eigenschaft der medizinischen Einrichtung, insbesondere einer Fluidbahn, eine einteilige oder mehrteilige Komponente, anhand welcher die Eigenschaft bestimmt oder gemessen werden kann. In solchen Ausführungsformen kann die Einrichtung jeweils wenigstens ein Marker sein, der optisch, mittels Durchstrahlung, oder mittels eines weiteren Verfahrens zum Nachweis seiner Existenz und/oder Position erkennbar ist und insbesondere zu diesem Zweck vorgesehen ist. In solchen Ausführungsformen könnte man die Einrichtung als eine "passive" Einrichtung bezeichnen.

In einigen erfindungsgemäßen, beispielhaften Ausführungsformen ist die Einrichtung zum Ermitteln einer Eigenschaft der medizinischen Einrichtung eine Markierung, ein Marker, eine Spule, ein passives Element, ein Piezoelement, ein Element, das aus einem anderen Material als das Hartteil und/oder die Fluidbahn gefertigt ist, ein Element, das mit einer Spannungsquelle verbunden ist, ein Element, das mit einer Leiterbahn verbunden ist, und/oder eine beliebige Kombination hieraus.

In einigen beispielhaften erfindungsgemäßen Ausführungsformen wird unter einer Eigenschaft des medizinischen Fluids der Druck, unter dem es steht, der Fluss oder die Flussgeschwindigkeit, mit der es strömt, seine Temperatur, seine Leitfähigkeit, usw. verstanden, vorzugsweise jeweils z. B. zum Zeitpunkt der Messung durch den Wandler.

In einigen beispielhaften erfindungsgemäßen Ausführungsformen ist die Einrichtung zum Ermitteln einer Eigenschaft der medizinischen Einrichtung eine Einrichtung zum Einwirken auf das medizinische Fluid. Sie kann insbesondere ausgestaltet sein, um dieses zum Schwingen anzuregen, während das medizinische Fluid in einer der Fluidbahnen - und vorzugsweise in einer solchen, die mit dem Wandler in Verbindung steht - vorliegt.

In einigen beispielhaften erfindungsgemäßen Ausführungsformen ist die Einrichtung zum Ermitteln einer Eigenschaft der medizinischen Einrichtung wenigstens ein Piezoelement oder umfasst wenigstens ein solches.

Das Piezoelement kann ein Plastikpiezoelement sein oder umfassen.

In einigen beispielhaften erfindungsgemäßen Ausführungsformen sind wenigstens ein Abschnitt des Wandlers und/oder wenigstens ein Abschnitt der Einrichtung zum Ermitteln einer Eigenschaft der medizinischen Einrichtung mittels wenigstens eines additiven Aufbringungsverfahrens auf das Hartteil aufgebracht.

Insbesondere können unter einem additiven Aufbringungsverfahren die folgenden Verfahren verstanden werden:

- selektives Laserschmelzen (SLM),
- selektives Lasersintern (SLS),
- Selective Heat Sintering (SHS),
- Binder Jetting (Verfestigen von Pulvermaterial mittels Binder),
- Elektronenstrahlschmelzen (Electron Beam Melting = EBM)
- Fused Deposition Modelling (FDM oder auch Fused Filament Fabrication (FFF)),
- Auftragschweißen bzw. Cladding,
- Wax Deposition Modelling (WDM),
- Contour Crafting,
- Metall-Pulver-Auftragsverfahren (MPA),
- Kaltgasspritzen,
- Stereolithografie (SLA) + Mikro-SLA,
- Verfahren, welche Digital Light Processing (DLP) zur Belichtung nutzen,
- Liquid Composite Moulding (LCM),
- Laminated Object Modelling (LOM),
- 3D-Siebdruck von Metallen und
- Lichtgesteuerte Elektrophoretische Abscheidung.

In einigen beispielhaften erfindungsgemäßen Ausführungsformen umfasst das Aufbringungsverfahren ein maskierungsfreies Aufbringen oder besteht hieraus. Dies kann auf das erste, das zweite und/oder weitere Aufbringungsverfahren zutreffen, sowie auf Kombinationen hiervon.

In einigen beispielhaften erfindungsgemäßen Ausführungsformen ist unter einem maskierungsfreien Aufbringen ein Aufbringen ohne Maske oder Schablone zu verstehen.

In einigen beispielhaften erfindungsgemäßen Ausführungsformen ist das Aufbringungsverfahren kein Maskentintendruck, kein "ink stencil printing", kein "screen printing", kein Fotolithographie-Verfahren, insbesondere jeweils nicht in einem kontinuierlichen Verfahren.

In einigen beispielhaften, erfindungsgemäßen Ausführungsformen ist unter einem maskierungsfreien Aufbringen ein Verzicht auf jede - anschließend zu entfernende - Hilfsschicht oder Blende zu verstehen.

Unter "Maskierung" kann hierin ein Abschatten oder Schützen in geeigneter Form von Bereichen, welche nicht beschichtet werden dürfen, verstanden werden. Diese Maskierung geschieht z. B. durch Blenden (z. B. Siebdruck, Sprühlackierung, etc.) oder durch "Lack"-Masken (z. B. Wafer, Platinen-Fertigung, etc.).

In einigen beispielhaften erfindungsgemäßen Ausführungsformen weist die medizinische Einrichtung eine Vielzahl von Wandlern, d. h. zwei oder mehr, auf, welche, jeweils zumindest in einem Abschnitt hiervon, mittels eines additiven Aufbringungsverfahrens (oder mehrerer additiver Aufbringungsverfahren), vorzugsweise Druckverfahren(s), - direkt oder indirekt - auf das Hartteil aufgebracht sind oder wurden.

In einigen beispielhaften erfindungsgemäßen Ausführungsformen umfasst das Aufbringungsverfahren das Aufbringen von leitfähiger Tinte oder besteht hieraus.

Unter leitfähiger Tinte sind hier Fluide mit beispielsweise Nano- oder Mikropartikeln zu verstehen, die, wenn sie geeignet dicht zusammen aufgebracht werden, eine funktionale Bahn ergeben wie z. B. eine Leiterbahn (hierin auch als Elektrode bezeichnet), über die unter anderem Signale übertragen werden können. Im Sinne der Erfindung kommen aber auch andere Materialien und Aggregatszustände als leitfähige Tinte in Frage oder zählen hierzu. Leitfähige Tinte kann idealerweise auch biokompatibel sein, muss aber nicht. Es können ergänzend oder alternativ lebende Zellen, Proteine etc. Bestandteil der durch sie leitfähig gemachten Tinte sein.

Die leitfähige Tinte oder das alternative Material kann in beliebigen Aggregatszuständen verwendet werden. Unter einem Aggregatszustand im Sinne der vorliegenden Erfindung kann in einigen Ausführungsformen ein festes oder flüssiges Hydrogel verstanden werden, in welches ein Biomarker eingelagert ist. Es können auch gefrorene (also feste) Substanzen oder mikroinkapsulierte Wirkstoffe/Reagenzien verarbeitet werden. Auch können Bahnen per Sublimation/Kondensation aus der Gasphase abgeschieden werden.

Unter leitfähiger Tinte sind hier beispielsweise auch Liquide zu verstehen, die Karbon, leitende Polymere, Metallpartikel und/oder Kombinationen hiervon aufweisen, ferner metallisierte Tinte.

Die aus dem Stand der Technik bekannte Technik des Aerosoljet-Druckens (siehe hierzu die EP 2 559 656 A1) kommt hier als Beispiel für ein additives Aufbringen und insbesondere zum Bedrucken in Betracht. Die dort offenbarte Technik und die darin beschriebenen Vorrichtungen zum Ausführen dieser Technik eignen sich hierfür, insbesondere auch aufgrund der geometrischen Ausführung der Druckdüse (Nozzle tip), welche die notwendige Anzahl an Freiheitsgraden in der Bewegung beim Druckvorgang erlaubt.

Die vorliegende Erfindung ist natürlich nicht auf die Anwendung des Aerosoljets begrenzt. Der Fachmann erkennt, dass alle additiven und/oder maskierungsfreien Druckverfahren, insbesondere mittels welcher leitfähige Tinte im Sinne dieser Beschreibung appliziert werden kann, von der vorliegenden Erfindung umfasst sind.

In einigen erfindungsgemäßen, beispielhaften Ausführungsformen ist der wenigstens eine Wandler ein MID-Flusssensor (kurz für: Magneto-Induzierter Durchflusssensor).

In einigen erfindungsgemäßen, beispielhaften Ausführungsformen sind der wenigstens eine Wandler zum Messen oder Bestimmen von Leitfähigkeit, Druck, Fluss, Spannung oder Strom konfiguriert oder ausgestaltet. Der hierin als Signalwert bezeichnete Wert des Signals, das der Wandler ausgibt, kann somit ein Wert sein, der die Leitfähigkeit, den Druck, den Fluss, die Spannung, die Konzentration, den Strom oder eine andere Messgröße wiedergibt oder von diesem abhängt.

Ein Wert kann eine Zahlenangabe mit oder ohne Dimension sein.

In einigen erfindungsgemäßen, beispielhaften Ausführungsformen ist die medizinische Einrichtung eine Blutkassette, ein Blutschlauch, ein Blutfilter, ein Schlauch, ein Schlauchsystem, ein Schlauchset oder jeweils ein Teil hiervon.

In einigen erfindungsgemäßen, beispielhaften Ausführungsformen ist die medizinische Einrichtung ein Einwegprodukt oder ein Disposable.

In einigen erfindungsgemäßen, beispielhaften Ausführungsformen umfasst das Verfahren keine manuelle Kalibrierung, kein Verwenden eines Kalibrier- oder Eichmaßes, etwa in Form eines Referenzvolumens (am Beispiel eines Durchflusssensors).

In einigen erfindungsgemäßen, beispielhaften Ausführungsformen erfolgt der Schritt des Korrigierens und/oder das Verwenden der Relation zwischen dem ersten und dem zweiten Wert nur in dem Fall, dass eine Differenz, ein Unterschied, ein Verhältnis oder eine andere mathematische Beziehung zwischen dem ersten Wert und dem zweiten Wert wenigstens einen vorbestimmten Wert erreicht oder übersteigt.

Eine Relation kann ein mathematischer Bezug zwischen den in Relation zueinander stehenden Werten, z. B. Zahlenwerten, sein. Als Beispiele einer Relation sind der Quotient, das Produkt, eine Prozentangabe, die Größe einer Summe oder Differenz, das Fallen in dieselben oder in verschiedene vorbestimmte Klassen von Werten, das gemeinsame Liegen auf derselben Seite eines Schwellenwerts, usw. zu nennen.

In einigen, beispielhaften erfindungsgemäßen Ausführungsformen umfassen die mittels eines oder mehrerer additiver Aufbringungsverfahren aufgebrachten Abschnitte oder Komponenten zusätzlich zum wenigstens einen Wandler wenigstens Leiterbahnen, Elektroden, einen Multipolstecker oder jeweils eine Vielzahl hiervon.

In einigen beispielhaften, erfindungsgemäßen Ausführungsformen ist oder umfasst das additive Aufbringungsverfahren kein Kleben, Schrauben, Stecken, kein screen-printing, kein tampon printing und/oder verwendet keine Silberpaste, keine Aluminiumpaste, keine "inkjet-produced patterns of solid copper".

In einigen beispielhaften, erfindungsgemäßen Ausführungsformen hat oder ist der Sensor, oder der Wandler, keine Folie.

Ein "medizinisches Fluid" im Sinne der vorliegenden Erfindung schließt jede medizinische Flüssigkeit und/oder jedes medizinische Gas sowie beliebige Kombinationen ein. Vorzugsweise handelt es sich bei dem Fluid um Blut.

In einigen beispielhaften, erfindungsgemäßen Ausführungsformen trifft das für das medizinische Fluid Gesagte auch auf nicht-medizinische Fluide zu. Die vorliegende Erfindung ist daher nicht auf medizinische Fluide beschränkt.

Eine erfindungsgemäße medizinische Einrichtung kann eine Einmalkomponente oder ein Einmalartikel ("Disposable") sein, welcher beispielsweise aus einem Kunststoffmaterial gefertigt ist.

Die erfindungsgemäße medizinische Einrichtung oder ihr Hartteil können mittels eines Spritzgussverfahrens hergestellt sein.

Die erfindungsgemäße medizinische Einrichtung kann über Flüssigkeits- und/oder Gasanschlüsse, über halboffene Kanäle und/oder Kammern verfügen. Ein oder mehrere Abdeckungselemente, wie beispielsweise Membranen oder Folien, können für den Abschluss und/oder die Abdichtung der Kanäle und Kammern sorgen.

Die Blutbehandlung, für welche die medizinische Einrichtung bestimmungsgemäß verwendet wird, kann beispielsweise ein Dialysierverfahren, eine Hämodialyse, Hämofiltration, Hämodiafiltration und/oder dergleichen, sein.

In bestimmten beispielhaften erfindungsgemäßen Ausführungsformen ist die medizinische Einrichtung eine Blutkassette. Das Hartteil ist in diesen Fällen ein Kassettenkörper oder Kassettengrundkörper oder ein Schlauchabschnitt.

In bestimmten beispielhaften erfindungsgemäßen Ausführungsformen weist die medizinische Einrichtung ferner wenigstens zwei Konnektoren für Pumpschlauchsegmente für peristaltische Pumpen, mit oder ohne die Pumpschlauchsegmente, auf.

In bestimmten beispielhaften erfindungsgemäßen Ausführungsformen weist die medizinische Einrichtung eine Single-Needle-Sterilmembran auf.

In einigen beispielhaften, erfindungsgemäßen Ausführungsformen trifft das für die medizinische Einrichtung Gesagte auch auf nicht-medizinische Einrichtungen zu. Die vorliegende Erfindung ist daher nicht auf medizinische Einrichtungen beschränkt.

In bestimmten beispielhaften erfindungsgemäßen Ausführungsformen ist das Piezoelement ein PVDF ("Plastikpiezo").

In bestimmten beispielhaften erfindungsgemäßen Ausführungsformen ist das Piezoelement mittels eines der hierin genannten Aufbringungsverfahren auf die medizinische Einrichtung aufgebracht.

In bestimmten beispielhaften erfindungsgemäßen Ausführungsformen ist das Piezoelement, z. B. als PVDF-Element, mittels Aerosoljet auf die medizinische Einrichtung aufgebracht.

In bestimmten beispielhaften erfindungsgemäßen Ausführungsformen ist das Piezoelement an der Innenseite der Wand der Messzelle angebracht.

In bestimmten beispielhaften erfindungsgemäßen Ausführungsformen ist das Piezoelement an der Außenseite dieser Wand angebracht.

In bestimmten beispielhaften erfindungsgemäßen Ausführungsformen dient das Piezoelement sowohl als Aktuator zur Anregung der Schwingungen als auch als Sensor, d. h. es nimmt alternierend die Reflektion der zuvor angeregten Schwingungen auf und wandelt sie in elektrische Spannung um, die dann ausgewertet werden kann. Durch die Messung der zeitlichen Abstände der reflektierten Signale kann auf die Größe der Messzelle geschlossen werden.

In bestimmten beispielhaften erfindungsgemäßen Ausführungsformen ist ein weiteres Piezoelement an der gegenüberliegenden Wand angebracht. Auch hier ist die Positionierung sowohl an der Außenseite als auch an der Innenseite der Wand möglich. In dieser Ausführungsform dient das erste Piezoelement als Aktor und das zweite Piezoelement als Sensor. Die Transmissionszeit oder die Laufzeit der angeregten Schwingungen wird bestimmt. Aus ihr kann auf die Eigenschaft der Einrichtung geschlossen werden. In einer Ausführungsform dienen die beiden Piezoelemente hierbei jeweils alternierend als Emitter und als Sensor.

In bestimmten beispielhaften erfindungsgemäßen Ausführungsformen ist die Einrichtung gemäß Anspruch 1 keine medizinische Einrichtung, die Vorrichtung mit der Einrichtung zum Aufnehmen oder Ankoppeln der Einrichtung keine Blutbehandlungsvorrichtung. Die vorliegende Erfindung ist somit nicht auf ihren Einsatz in der Medizintechnik beschränkt.

In bestimmten beispielhaften, erfindungsgemäßen Ausführungsformen ist die Behandlungsvorrichtung ausgestaltet als Blutbehandlungsvorrichtung, insbesondere als Apheresevorrichtung oder Dialysevorrichtung, insbesondere als Hämofiltrationsvorrichtung, als Hämodiafiltrationsvorrichtung, als Filtrationsvorrichtung oder als Vorrichtung zum extrakorporalen Gasaustausch.

In bestimmten beispielhaften, erfindungsgemäßen Ausführungsformen ist wenigstens eine Einrichtung vorgesehen, die zum Ausführen eines hierin genannten Verfahrensschritts vorgesehen, bestimmt und/oder programmiert ist. Solche Einrichtungen kann für jeden hierin genannten Verfahrensschritt vorgesehen sein. Die jeweilige Einrichtung kann jeweils z. B. Teil einer beliebigen der erfindungsgemäßen Vorrichtungen sein oder hiermit in Signalverbindung verbunden sein.

Einige oder alle erfindungsgemäßen Ausführungsformen können einen oder mehrere der oben oder im Folgenden genannten Vorteile aufweisen.

Moderne medizinische Systeme zur Blutbehandlung weisen üblicherweise eine Blutbehandlungsvorrichtung (auf der sogenannten "Maschinenseite") und daran anbringbare medizinische Einrichtungen (auf der sogenannten "Einrichtungsseite") auf. Ist die medizinische Einrichtung ein Einwegartikel oder Disposable, so ist hierin auch von der "Disposableseite" die Rede. Obgleich die vorliegende Erfindung nicht auf Disposables beschränkt ist, wird hierin exemplarisch für medizinische Einrichtungen auch auf Disposables Bezug genommen, ohne dass dies beschränkend zu verstehen ist. Die zu einer Blutbehandlungssitzung verwendeten Einwegartikel werden aus Gründen der Hygiene nach der Blutbehandlungssitzung entsorgt, da sie im Kontakt mit Blut des Patienten standen oder gestanden haben können.

Zur Überwachung der Behandlung sind Sensoren und Aktoren erforderlich, die auf dem Einwegartikel bzw. der medizinischen Einrichtung Parameter messen bzw. auf die Einrichtung einwirken.

Mittels Spritzguss günstig hergestellte Disposables unterliegen in der Regel gewissen Fertigungstoleranzen, welche der absoluten Genauigkeit der geometrischen oder räumlichen Eigenschaften oder Abmessungen der Disposables abträglich sind.

Medizinische Disposables weisen häufig Messzellen auf, deren genaue Abmessungen Einfluss auf hierin vorgenommene Messungen haben. Dies gilt im besonderen Maße für funktionalisierte oder teilfunktionalisierte Disposables, die Sensoren oder Wandler tragen, aber auch beim Einwirken auf medizinische Disposables.

Auch beim Einwirken mittels Aktuatoren auf medizinische Disposables sind Situationen bekannt, in denen die genauen Abmessungen des Disposables oder eines konkreten Abschnitts hiervon Einfluss auf die Steuerung oder den Antrieb eines Aktuators haben.

Im Stand der Technik ist bekannt, solche medizinische Disposables durch individuelle Messung ab Werk zu kalibrieren. Hierzu wird unmittelbar nach der Produktion jede individuelle Messzelle untersucht, z. B. durch räumliche Vermessung oder Volumenbestimmung o. ä.. Die Messdaten werden anschließend mit dem Produkt, z. B. dem medizinischen Disposable, ausgeliefert, z. B. auf einem Speichermedium gespeichert. Mit diesen Messdaten kann das medizinische Disposable dann am Ort der Nutzung bzw. an der Behandlungsvorrichtung, welche mit dem medizinischen Disposable aufgerüstet wurde, kalibriert werden. Dieses Verfahren weist jedoch einige Nachteile auf. Zum einen erfolgt keine Korrektur für Geometrie- oder Materialveränderungen wie z. B. Verformungen durch Fallenlassen, Sonneneinwirkung, usw., die nach der o. g. initialen Vermessung eintreten. Weiterhin ist das Verfahren logistisch aufwändig und erfordert mit dem Kalibrierschritt einen weiteren Verfahrensschritt am Ort der Produktion des medizinischen Disposables, was dessen Fertigungskosten erhöht.

Aufgrund der Fertigungstoleranzen eines medizinischen Einwegartikels kann zudem keine einmalige, allgemeine Kalibrierung für alle betroffenen Komponenten bzw. Einwegartikel eines Typs erfolgen. Die absolute Kalibrierung basierend auf solchen allgemeinen Daten führt regelmäßig zu einer zu ungenauen Messung im Falle eines Sensors bzw. einer zu ungenauen Steuerung im Falle eines Aktuators. Somit ist es erschwerend zudem erforderlich, jede betroffene Komponente individuell zu kalibrieren.

Ein Beispiel für einen Sensor, dessen Messergebnis signifikant von der genauen Kenntnis der räumlichen Größe seiner Messzelle abhängt, ist etwa ein magnetoinduktiver Durchflusssensor (MID). Hierbei hängen die Messergebnisse direkt von der Größe der Querschnittsfläche der Fluidbahn im Bereich der Messelektroden ab. Ein Beispiel im Bereich der Aktuatoren stellt etwa der Antrieb einer magnetisch getriebenen und/oder magnetisch gelagerten Impellerpumpe im medizinischen Disposable dar.

Hier kann die vorliegende Erfindung vorteilhaft Abhilfe schaffen. Erfindungsgemäß kann die Auswirkung einer nach Verlassen des Ortes der Herstellung erfolgte Veränderung etwa der Geometrie des Disposables auf ein Messsignal kompensiert werden.

Der Fachmann erkennt jedoch, dass die Anwendung der Erfindung nicht auf diese Beispiele begrenzt ist, sondern alle Arten von Sensoren oder Aktuatoren in oder auf Substratkörpern betrifft, welche Fertigungstoleranzen unterliegen und deren genaue räumliche Maße deshalb a priori nicht ausreichend bekannt sind. Solche Anwendungen sind zwar insbesondere bei medizinischen Verfahren und den dort verwendeten Einwegartikeln vorteilhaft anwendbar, jedoch ist die Erfindung nicht auf das Gebiet der Medizintechnik beschränkt.

Die weiteren Vorteile umfassen die Folgenden: Eine Funktionalisierung der Einwegartikel selbst, also die Integration der gesamten zum Überwachen eines konkreten Parameters jeweils erforderlichen Sensorik in den Einwegartikeln, hat sich bisher zumeist als wirtschaftlich unrentabel herausgestellt, da die vergleichsweise teuren Sensorbauteile nach jeder Verwendung verworfen werden würden. Deshalb sind herkömmliche Einwegartikel zur medizinischen Blutbehandlung zumeist sehr einfach gestaltet, bestehen im Wesentlichen aus dem Schlauch zur Durchleitung des Bluts. Sensoren tragen sie aus dem vorstehend genannten Grund i. a. R. nicht.

Diese Sensoren und Aktoren sind im Stand der Technik folglich weitgehend maschinenseitig vorgesehen. Von dort wirken sie über eine Vielzahl von Sensor- und/oder Aktuatorschnittstellen auf die medizinische Einrichtung ein oder stehen mit dieser in Wechselwirkung. Diese Schnittstellen bestimmen in der Regel den Formfaktor und führen dazu, dass medizinische Systeme des Standes der Technik nicht beliebig klein ausgeführt werden können.

Die vorliegende Erfindung erlaubt es nun hingegen erstmals vorteilhaft, eine kostengünstige und damit wirtschaftliche Alternative zur Funktionalisierung von medizinischen Einrichtungen und auch Einwegartikeln bereit zu stellen. Sie erlaubt damit vorteilhaft die Miniaturisierung der eingesetzten Systeme, z. B. Blutbehandlungssysteme. Deren Miniaturisierung ist nicht zuletzt deshalb wünschenswert, da dies zur Reduzierung sowohl der extrakorporalen Blutmenge als auch des Aufwands für die extrakorporale Förderung und Hydraulik beiträgt.

Im Gegensatz zum Stand der Technik, in dem die Funktionalisierung durch die Integration der individuellen Sensorkomponenten in die Einwegartikel mit den vorstehend genannten Nachteilen versucht wurde, stellt die Erfindung wie vorstehend beschrieben eine Lösung zur wenig aufwendigen Verlagerung von Sensorkomponenten auf die medizinische Einrichtung dar.

Vorteilhaft ist ferner, dass mit der vorliegenden Erfindung eine Methode zur Verfügung gestellt wird, bei der alle einrichtungsseitig angeordneten Sensorkomponenten mit demselben Produktionsverfahren integriert werden können, was den Fertigungsaufwand weiter senken kann.

Die vorliegende Erfindung erlaubt dabei wahlweise eine vollständige Funktionalisierung oder eine nur teilweise Funktionalisierung der erfindungsgemäßen Einrichtung, die aufgrund der besonderen Art, wie die Wandler auf die Einrichtung verlagert werden, ohne nennenswerten wirtschaftlichen Verlust gemeinsam mit den Wandlern verworfen werden kann.

Unter einer teilweisen Funktionalisierung wird hierin beispielsweise im Falle eines Wandlers verstanden, dass die Kernaufgabe des Wandlers, also das Erfassen einer physikalischen, chemischen oder anderen Größe sowie das Wandeln dieser Größe in eine elektromagnetisch übertragbare Substitutgröße, auf der medizinischen Einrichtung selbst, entweder invasiv (d. h. im Kontakt mit Blut oder einer Behandlungsflüssigkeit) oder nicht invasiv geschieht, jedoch z. B. die Verarbeitung des Signals sowie die Interpretation und weitere Schritte, die höherwertige z.B. integrierte elektronische Bauteile erfordert, auf Maschinenseite erfolgt. Ein Teil oder die gesamte Verarbeitung des Signals kann in einigen Fällen jedoch auch komplett auf der Einrichtungsseite erfolgen. Bei letzterer Ausführungsform der vorliegenden Erfindung wird von einer vollständigen Funktionalisierung gesprochen: wenigstens eine Sensoranordnung oder ein Sensor liegt vollständig additiv auf die Einrichtung aufgebracht auf letzterer vor. Dabei können die einrichtungsseitigen Komponenten einer Sensoranordnung oder eines Sensors erfindungsgemäß alle durch dasselbe maskenfreie und additive Produktionsverfahren aufgebracht werden, wie vorstehend ausgeführt.

Von Vorteil ist ferner, dass, wie erfindungsgemäß auch vorgeschlagen, Einwegartikel, wenn sie mit individuellen vollständigen Sensoren ausgestattet werden, somit vollständig funktionalisiert werden können. Das hierin vorstellte Verfahren zum Aufbringen von Wandlern oder anderen Komponenten von Sensoren macht dies möglich.

Die teilweise Funktionalisierung und damit verbunden, der Verbleib der komplexen, zur Nachverarbeitung und Auswertung der Signale notwendigen Elektronik auf der Maschinenseite ist dadurch vorteilhaft, dass nur die zumeist einfacher gestalteten Komponenten der jeweiligen Sensoren auf der Einrichtung angeordnet sind, die durch die gemeinsame additive und maskierungsfreie Drucktechnik angebracht werden.

Die Funktionalisierung ist ferner wirtschaftlich von Vorteil, da die Herstellungskosten durch die gemeinsame Verwendung derselben Produktionstechnologie, idealerweise im selben Produktionsschritt für alle einrichtungsseitig angeordneten Sensorkomponenten, äußerst kostengünstig erfolgen kann.

Da das Aufbringungsverfahren vorzugsweise ein additives, maskierungsfreies Druckverfahren ist, kann eine veränderte Version der Einrichtung, beispielsweise mit neuen Sensorgeometrien, optional sogar allein durch neues Aufspielen des entsprechenden Datensatzes in der Software produziert werden. Es ist keine Veränderung an der Produktionshardware notwendig, wie beispielsweise die Neuanschaffung eines Spritzgusswerkzeugs, das in der Produktion der medizinischen Einrichtung mittels Spritzguss erforderlich wäre. Auch dieser Aspekt führt zu einer vereinfachten Fertigung und zu verbesserter Wirtschaftlichkeit.

Ein weiterer Vorteil der Erfindung zu herkömmlichen (nicht-funktionalisierten) Systemen besteht darin, dass die zu messende physikalische oder chemische Größe nicht mechanisch auf die Maschinenseite geführt werden muss, wie das beispielsweise bei bekannten Drucksensoren der Fall ist, bei denen die zu messenden Druckverhältnisse über eine pneumatische Leitung zu einer Messmembran geführt werden, welche nicht disposable ist und maschinenseitig angeordnet ist. Um diese vor Flüssigkeit zu schützen sind regelmäßig aufwändige Schutzmembranen notwendig, welche als Transducerprotector (TP) bezeichnet werden. Erfindungsgemäß können also alle mechanischen Schnittstellen zur Maschine entfallen. Alle zu messenden Größen können mittels der integrierten Teile der Sensorik zumindest in analoge elektromagnetische Signale (Strom, Spannung, optische Signale) gewandelt und erst in dieser Form zur Verarbeitungs- und Auswerteeinheit auf der Maschinenseite übermittelt werden. Zur Ankopplung der gesamten einrichtungsseitigen Sensorik ist nur noch eine entsprechende mehrkanalige elektro-magnetische und/oder optische Schnittstelle notwendig (Multistecker oder Multipolstecker), was eine signifikante Verkleinerung derselben erlaubt.

Die vorliegende Erfindung erlaubt ferner eine optimale Übermittlung des Signals vom Wandler des Sensors an z. B einen Multistecker durch Unterscheidung verschiedener Anschluss- bzw. Leitungssequenzen und der Verwendung der bezüglich Leitungssicherheit und Produktionsgeschwindigkeit und Kosten optimalen Technik zum Aufbringen der Leiterbahnen. Weiterhin stellt die Erfindung die optimale Konnektierung zwischen den einzelnen Leitungssequenzen zur Verfügung.

Im Folgenden wird die vorliegende Erfindung unter Bezugnahme auf die beigefügte, stark vereinfachte Zeichnung beschrieben. In der Zeichnung bezeichnen identische Bezugszeichen gleiche oder identische Elemente. Es zeigen:

Fig. 1    ein medizinisches System zur Blutbehandlung mit einer medizinischen Einrichtung gemäß der vorliegenden Erfindung als teilfunktionalisiertes Disposable;

Fig. 2    eine medizinische Einrichtung gemäß einer zweiten beispielhaften Ausführungsform der vorliegenden Erfindung;

Fig. 3 einen Ausschnitt des fluidführenden Kanals oder Schlauchs des Hartteils der Einrichtung mit einer Einrichtung zum Ermitteln einer Eigenschaft der medizinischen Einrichtung, insbesondere der Fluidbahn, in einer ersten Ausführungsform;

Fig. 4 einen Ausschnitt des fluidführenden Kanals oder Schlauchs des Hartteils der Einrichtung mit einer Einrichtung zum Ermitteln einer Eigenschaft der medizinischen Einrichtung, insbesondere der Fluidbahn, in einer zweiten Ausführungsform; und

Fig. 5 zeigt eine exemplarische Ausführungsform einer offenbarten Auswerteeinheit.

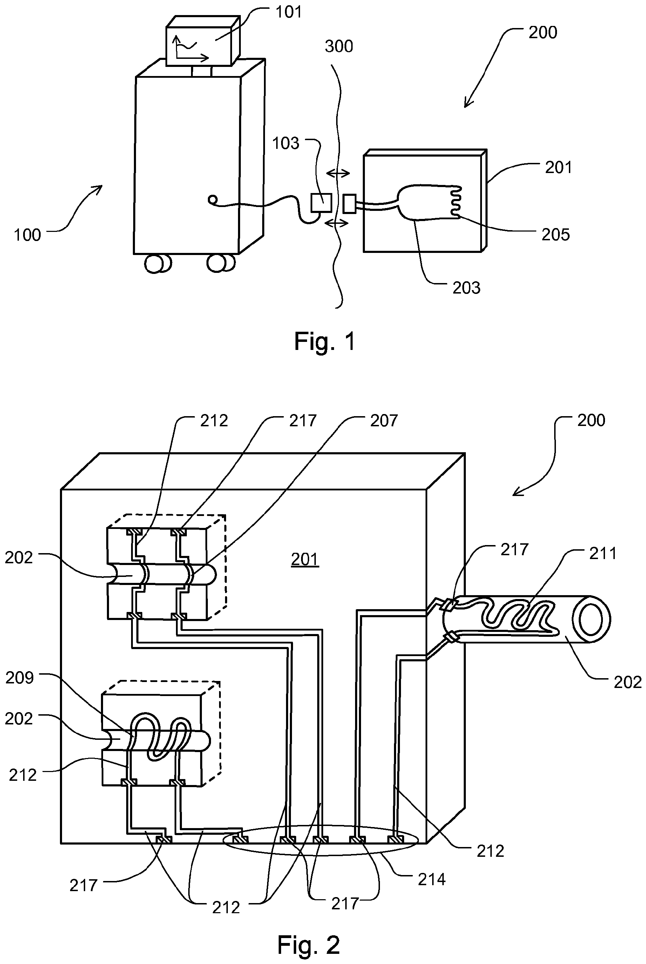

Fig. 1 zeigt sehr schematisch und nur beispielhaft eine erfindungsgemäße Blutbehandlungsvorrichtung 100 als Beispiel einer erfindungsgemäßen Behandlungsvorrichtung, mit einer erfindungsgemäßen medizinischen Einrichtung 200 (kurz: Einrichtung 200) als Beispiel eines erfindungsgemäßen Einmalartikels oder Disposables.

Die Einrichtung 200 ist exemplarisch als ein Disposable ausgestaltet. Sie wurde rein optional mittels eines additiven, maskierungsfreien Druckverfahrens teilfunktionalisiert.

Die Blutbehandlungsvorrichtung 100 und die erfindungsgemäße Einrichtung 200 sind über eine Schnittstelle 300 miteinander in Signalkommunikation verbunden.

Die Einrichtung 200 weist ein Hartteil 201 auf. Auf dem Hartteil 201 sind Teile einer Sensoranordnung vorgesehen, hier eine Leiterbahn 203 und ein Wandler 205.

Der Wandler 205 kann beispielsweise ein Druckaufnehmer sein. Er kann auf das Hartteil 201 aufgedruckt sein. Er kann disposableseitig lediglich die zu messende Größe Druck in ein analoges elektrisches Signal wandeln.

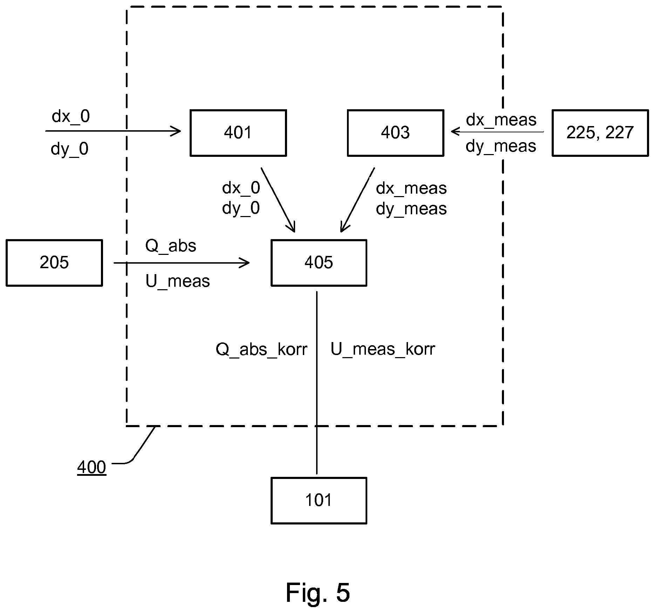

Das elektrische Signal wird über die - ebenfalls optional additiv aufgedruckte - Leiterbahn 203 zu der definierten Schnittstelle 300 geleitet, welche in Verbindung mit einer maschinenseitigen Auswerteeinheit 400 (siehe Fig. 5) steht. Die Auswerteeinheit 400, welche zu Fig. 5 weiter ausgeführt ist, ist in Fig. 1 angedeutet durch einen Monitor 101 zur Darstellung der mit ihr erzielten Auswerteergebnisse.

Maschinenseitig kann das Signal mittels eines AD (Analog-Digital-)-Wandlers oder AD-Converters (kurz: ADC) 103 digitalisiert werden, es können Schritte der Nachverarbeitung (Filter, Glättung, Fouriertransformation, Zero-Filling etc.) erfolgen, bevor schließlich eine Auswertung und Interpretation erfolgt. Alle diese optionalen Schritte können z. B. in der Auswerteeinheit 400 durchgeführt werden.

**Fig. 2** zeigt eine medizinische Einrichtung 200 gemäß einer zweiten beispielhaften Ausführungsform der vorliegenden Erfindung.

Die medizinische Einrichtung 200 ist hier wiederum ein teilfunktionalisiertes Disposable, mit drei unterschiedlichen Wandlern 207, 209 und 211.

Dabei zeigt Fig. 2 wie auch die folgenden Figuren je ein Beispiel von aufgebrachten Wandlern 207, 209 und 211 einerseits und Leiterbahnen 212 andererseits, wobei Wandler und Leiterbahn mittels verschiedener Aufbringungsverfahren aufgebracht wurden oder erfindungsgemäß aufgebracht worden sein können - alternativ können sowohl wenigstens einer der Wandler 207, 209 und 211 einerseits und eine der Leiterbahnen 212 andererseits jeweils mittels mehr als einem Aufbringungsverfahren - sondern wenigstens mittels eines ersten und eines zweiten Aufbringungsverfahrens - aufgebracht worden sein.

Oben links ist in Fig. 2 beispielhaft ein Wandler 207 zur Messung der Leitfähigkeit angeordnet. In der vorliegenden, vereinfachten Darstellung besteht dieser aus zwei Leiterbahnen 212, welche erfindungsgemäß mittels des additiven, maskierungsfreien Druckverfahrens im Inneren eines fluidführenden Kanals 202 der Einrichtung 200 aufgebracht sind oder wurden.

Die beiden anderen Wandler 209, 211 zeigen beispielhafte Ausführungen von Druckaufnehmern. Diese können als Dehnungsmessstreifen an der Innenkontur (siehe den Wandler 209) oder an der Außenkontur (siehe den Wandler 211) des fluidführenden Kanals oder Schlauchs 202 der Einrichtung 200 angebracht sein.

Über die Wandler 209, 211 hinaus zeigt Fig. 2 beispielhaft die elektro-magnetische Signalführung mit Leiterbahnen 212 und Kontakte 217 zur Maschinenschnittstelle 300, hier beispielhaft einen Multipolstecker 214. Die Leiterbahnen 212 zur Signalleitung können hierbei mit demselben additiven und maskierungsfreien Verfahren, insbesondere im selben Verfahrensschritt, auf die planare Fläche des Hartteils 201 aufgebracht werden oder worden sein.

Es sind aber auch nicht-planare (dreidimensionale) Leitungsführungen im oben beschriebenen Sinn oder Kreuzungen von (entsprechend isolierten) Leitungen möglich.

Die Leiterbahnen 212 sowie die Kontakte 217 können auch mittels einer zweiten, ebenfalls additiven und maskierungsfreien Drucktechnik aufgebracht werden, z. B. in einem zweiten Produktionsschritt, der dem Aufbringen der Wandler 207, 209, 211 folgt. Die Gesamtheit der Leiterbahnen 212 zur Signalführung vom Ort des jeweiligen Wandlers 207, 209, 211, welcher die zu messende Größe z. B. in ein elektromagnetisches Signal wandelt, das mittels der Leiterbahnen 212 zur Maschinenschnittstelle 300 geführt wird, kann auch einzelne Teile aus leitfähigen Polymeren beinhalten, die mittels Zweikomponenten-Spritzguss aufgebracht sind, werden oder wurden.

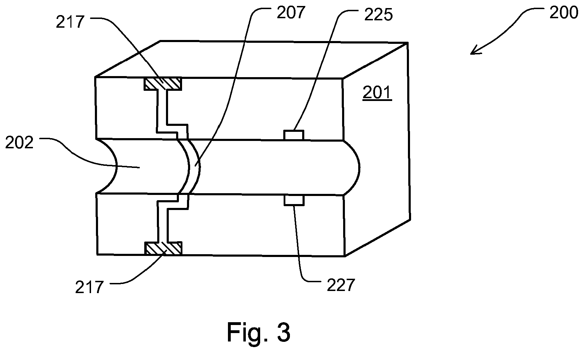

**Fig. 3** zeigt einen Ausschnitt des fluidführenden Kanals oder Schlauchs 202 des Hartteils 201 der Einrichtung 200 mit einer Einrichtung zum Ermitteln einer Eigenschaft, hier einer geometrischen Eigenschaft, der medizinischen Einrichtung 200, insbesondere der Fluidbahn 202, in einer ersten Ausführungsform.

Der Wandler 207 kann Teil eines MID-Flusssensors sein, der den Fluss durch den Kanal 202 misst.

Der Kanal 202 kann im Bereich des Wandlers 207 eine kreisrunde, rechtwinklige oder andere Querschnittsform oder Grundform aufweisen. Dies gilt insbesondere für den Bereich, in dem der Wandler 207 vorliegt, also für die Messzelle.

Die Einrichtung 200 weist in dieser exemplarischen Ausführungsform zumindest zwei Piezoelemente oder Elektromagnete 225 und 227 auf. Diese können als Beispiel für eine Einrichtung zum Ermitteln einer Eigenschaft der medizinischen Einrichtung 200 oder eines Teils hiervon verstanden werden. Die Piezoelemente oder Elektromagnete 225 und 227 sind hier optional als Spulen ausgestaltet. Die Spulen liegen optional auf oder an gegenüberliegenden Seiten der Messzelle.

Die Einrichtung zum Ermitteln einer Eigenschaft der medizinischen Einrichtung 200 kann in jeder erfindungsgemäßen Ausführungsform Teil des Wandlers 207 sein, sie kann alternativ unabhängig von diesem vorliegen.

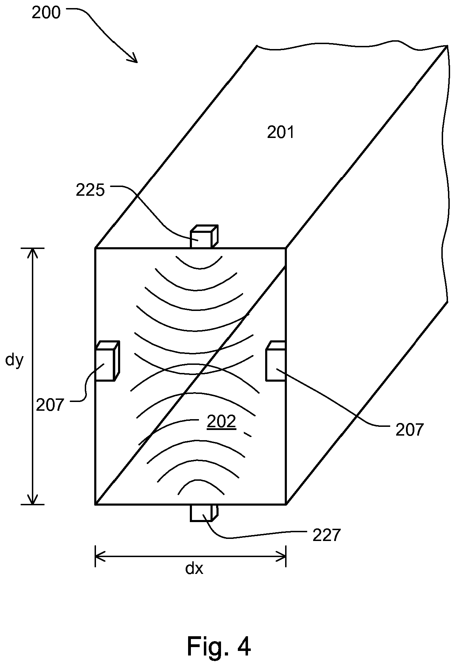

**Fig. 4** zeigt einen Blick hinein in einen Ausschnitt des fluidführenden Kanals oder Schlauchs 202 des Hartteils 201 der Einrichtung 200 (hier exemplarisch ein Disposable) in einer zweiten Ausführungsform. Die Einrichtung 200 weist eine Einrichtung zum Ermitteln einer (hier exemplarisch: geometrischen) Eigenschaft der medizinischen Einrichtung 200, insbesondere der Fluidbahn 202, auf.

Der Kanal 202 hat hier exemplarisch eine rechteckige Querschnittsform oder Grundform, jedenfalls im Bereich der Messzelle.

Die Einrichtung 200 weist in dieser exemplarischen Ausführungsform unverändert zumindest zwei Piezoelemente oder Elektromagnete 225 und 227 auf. Diese sind hier wiederum optional als Spulen ausgestaltet. Die Spulen liegen optional auf oder an gegenüberliegenden Seiten der Messzelle.

Erfindungsgemäß sind diese Spulen 225 und 227 auf der Außenseite der Messzelle angeordnet, derart, dass die Messzelle von einem größtenteils homogenen Magnetfeld durchsetzt wird. Senkrecht zur Verbindungsachse der beiden Spulen 225 und 227 (Magnetfeldrichtung) sind die Messelektroden des Wandlers 207 angeordnet. Diese sind invasiv positioniert, d. h. sie stehen in direktem Kontakt mit der zu messenden Flüssigkeit. Hierzu reichen sie durch die Kanalwand hindurch in die Fluidbahn 202 hinein oder sind innen auf die Kanalwand der Fluidbahn 202 aufgedruckt.

In der - exemplarisch maschinenseitig, optional alternativ einrichtungsseitig, vorgesehenen - Auswerteeinheit 400 (siehe Fig. 5) ist im Beispiel der Fig. 4 eine Funktion zum Ermitteln einer Eigenschaft des medizinischen Fluids in Abhängigkeit von einem Signalwert, der mittels des Wandlers 207 ermittelt wird, hinterlegt. Alternativ kann die Auswerteeinheit 400 hierauf zurückgreifen. Die vorgestehend genannte Funktion kann eine generische, typspezifische Kalibration oder Kalibrierung sein. Dies kann spezifisch für die entsprechende Wandler-Einrichtung/Disposable-Kombination sein. Im vorliegenden Beispiel stellt Kalibration im Wesentlichen eine Funktion dar, die für den jeweiligen Messwert, d. h. die abgegriffene Spannung an den Messelektroden des Wandlers 207 den vorliegenden Fluss angibt:

$$Q\_abs = f\ (U\_meas)$$

Die vorstehende, beispielhafte Kalibrierfunktion f ist unter anderem linear abhängig vom Querschnitt der Messzelle. Während Abweichungen von einem Sollwert, die durch Fertigung oder Handhabung bedingt sind, in der Achse der Messelektroden, also beim Durchmesser dx, senkrecht zum in Fig. 4 angedeuteten Magnetfeld die Genauigkeit der Kalibrierung nicht so stark beeinflussen, können Abweichungen von einem Sollwert dy_0 im Durchmesser dy, also in der Achse parallel zum Magnetfeld unkorrigiert zu einer signifikanten Störung der Absolutkalibrierung führen.

Die Erfindung kann deshalb für das hier gezeigte Beispiel der Fig. 4 vorsehen, dass der Abstand der Wände der Messzelle in y-Richtung mittels der in Fig. 5 gezeigten Einrichtung 403 zum Ermitteln der Eigenschaft der medizinischen Einrichtung wenigstens in der Achse des Magnetfelds vor Ort bzgl. ihrer Abweichung vom Sollabstand dy_0 verifiziert wird. Der Sollabstand kann als das Ergebnis einer Referenzmessung hinterlegt oder auslesbar sein.

Wenn eine Korrektur z. B. aufgrund der Differenz zwischen Sollabstand dy_0 für nötig befunden wird, so kann eine Korrektur der Kalibration bzgl. absoluter Flusswerte vorgenommen werden. Auf diese Weise kann ein Messergebnis mit der notwendigen Genauigkeit auch bei Messungen an kostengünstig im Spritzgussverfahren hergestellten Einwegartikeln, welche naturgemäß Fertigungstoleranzen unterliegen, sichergestellt werden.

Für diese Korrektur kann z. B. die o. g. Kalibrationsformel f mit dem Verhältnis aus tatsächlich gemessenem Durchmesser dy_meas als zweitem Wert und dem Solldurchmesser dy_0 des Fluidkanals als erstem Wert wie folgt multipliziert oder anderweitig berücksichtigt werden:

$$Q\_abs\_korr = f(U\_meas) * (dy\_meas/dy\_0)$$

Alternativ könnte das Signal U_meas geeignet korrigiert werden.

Zum Ermitteln des tatsächlichen Durchmessers dy_meas als zweiten Wert kann erfindungsgemäß wie im Beispiel der Fig. 4 ein Piezoelement 225, 227 zum Schwingen angeregt werden.

**Fig. 5** zeigt eine exemplarische Ausführungsform einer offenbarten Auswerteeinheit 400, welche programmiert und/oder konfiguriert ist zum Ausführen des erfindungsgemäßen Verfahrens.

Die Auswerteeinheit 400 kann Teil der erfindungsgemäßen Blutbehandlungsvorrichtung 100 sein. Sie kann alternativ unabhängig von dieser vorgesehen sein. Die im Folgenden diskutierten Einrichtungen zum Ausführen des erfindungsgemäßen Verfahrens sind im Beispiel der Fig. 5 Teil der Auswerteeinheit 400. Sie könnten alternativ Teil der Blutbehandlungsvorrichtung 100 oder einer anderen Einheit sein, womit nicht die Auswerteeinheit 400 gemeint ist.

Fig. 5 zeigt eine Einrichtung 401 zum Bereitstellen des Ergebnisses einer Referenzmessung oder-bestimmung für die geometrische Eigenschaft dx, dy als einen "ersten Wert". Das Ergebnis der Referenzmessung kann hierin beispielsweise die Erstreckung in x-Richtung und/oder in y-Richtung sein. Das Ergebnis ist in Fig. 5 daher nicht-beschränkend als dx_0 bzw. dy_0 bezeichnet. Der erste Wert kann in der Einrichtung 401 gespeichert sein, er kann alternativ in einer weiteren Einrichtung innerhalb oder außerhalb der Auswerteeinheit 400 gespeichert sein. Er kann alternativ bei Verwendung der medizinischen Einrichtung 200 von dieser ausgelesen werden, z. B. da er als Bar Code oder per Etikett auf der medizinischen Einrichtung 200 festgehalten ist.

Fig. 5 zeigt weiter eine Einrichtung 403 zum Empfangen eines Signals von der Einrichtung zum Ermitteln einer geometrischen Eigenschaft dx, dy der medizinischen Einrichtung 200, also z. B. von einem oder beiden der Piezoelemente 225, 227, wobei das Signal die geometrische Eigenschaft dx, dy betrifft. Das Signal geht als der "zweite Wert" in die weiteren Betrachtungen ein.

Fig. 5 zeigt ferner eine Einrichtung 405 zum Ausgeben eines Werts Q_abs_korr der Eigenschaft Q des medizinischen Fluids oder eines korrigierten Signalwerts U_meas_korr. Sowohl Wert Q_abs_korr als auch der korrigierte Signalwert U_meas_korr sind oder wurden unter Verwenden der Relation zwischen dem ersten Wert dx_0, dy_0 und dem zweiten Wert dx_meas, dy_meas ermittelt oder korrigiert.

Die Einrichtung 405 kann zum Anzeigen des Werts Q_abs_korr der Eigenschaft Q des medizinischen Fluids oder des korrigierten Signalwerts U_meas_korr mit dem Monitor 101 oder einer anderen Anzeigevorrichtung in Signalverbindung verbunden sein.

## Bezugszeichenliste

| | |
|---|---|
| 100 | Blutbehandlungsvorrichtung, Behandlungsvorrichtung |
| 101 | Monitor |
| 103 | AD-Wandler |
| 200 | Blutkassette als Beispiel einer medizinischen Einrichtung |
| 201 | Kassettenkörper oder Kassettengrundkörper, Hartteil; Hartkörper, Substrat |
| 202 | Fluidbahn, Kanal |
| 203 | Leiterbahn, Leiter oder Signalleiter |
| 205 | Wandler |
| 207 | Wandler |
| 209 | Wandler |
| 211 | DMS-Element als Wandler |
| 212 | Leiterbahn, Leiter oder Signalleiter |
| 214 | Multipolstecker |
| 217 | Kontakte |
| 225, 227 | Piezoelemente oder Elektromagnete |
| 300 | Schnittstelle, Maschinenschnittstelle |
| 400 | Auswerteeinheit |
| 401 | Einrichtung zum Bereitstellen des Ergebnisses einer Referenzmessung oder -bestimmung für die Eigenschaft |
| 403 | Einrichtung zum Empfangen eines Signals von der Einrichtung zum Ermitteln einer Eigenschaft (dx, dy) der medizinischen Einrichtung |
| 405 | Einrichtung zum Ausgeben eines Werts (Q_abs_korr) der Eigenschaft |

## Patentansprüche

1. Einrichtung (200), insbesondere eine medizinische Einrichtung, zu ihrer Aufnahme oder Ankoppelung an einer Behandlungsvorrichtung (100), wobei die Einrichtung (200) ausgestaltet ist mit jeweils wenigstens

   - einem Hartteil (201) mit Fluidbahnen (202) zum Führen eines, insbesondere medizinischen, Fluids, insbesondere Blut, durch das Hartteil (201);
   - einem Wandler (205, 207, 209, 211), wobei der Wandler (205, 207, 209, 211) angeordnet ist zum Messen einer Eigenschaft (Q) des Fluids, während dieses in einer der Fluidbahnen (202) vorliegt; und
   - einer Einrichtung zum Ermitteln einer, insbesondere geometrischen, Eigenschaft (dx, dy) der Einrichtung (200), insbesondere einer Fluidbahn (202) hiervon.

2. Einrichtung nach Anspruch 1, wobei die Einrichtung zum Ermitteln einer Eigenschaft (dx, dy) der Einrichtung (200) eine Einrichtung zum Einwirken auf das Fluid, insbesondere, um dieses zum Schwingen anzuregen, während das Fluid in einer der Fluidbahnen (202) vorliegt, ist oder umfasst.

3. Einrichtung nach einem der vorangegangenen Ansprüche, wobei die Einrichtung zum Ermitteln einer Eigenschaft (dx, dy) der Einrichtung (200) wenigstens ein Piezoelement (225, 227) ist oder umfasst.

4. Einrichtung nach Anspruch 3, wobei das Piezoelement (225, 227) ein Plastikpiezoelement ist oder umfasst.

5. Einrichtung nach einem der vorangegangenen Ansprüche, wobei wenigstens ein Abschnitt des Wandlers (205, 207, 209, 211) und/oder wenigstens ein Abschnitt der Einrichtung zum Ermitteln einer Eigenschaft (dx, dy) der Einrichtung (200) mittels wenigstens eines additiven Aufbringungsverfahrens aufgebracht sind oder wurden.

6. Einrichtung nach Anspruch 5, wobei das Aufbringungsverfahren ein maskierungsfreies Aufbringen ist oder umfasst.

7. Einrichtung nach Anspruch 5 oder 6, wobei das Aufbringungsverfahren ein Aufbringen von leitfähiger Tinte umfasst.

8. Einrichtung nach einem der vorangegangenen Ansprüche, wobei der wenigstens eine Wandler (205, 207, 209, 211) ein MID-Flusssensor ist.

9. Einrichtung nach einem der vorangegangenen Ansprüche, wobei der wenigstens eine Wandler (205, 207, 209, 211) zum Messen oder Bestimmen von Leitfähigkeit, Fluss, Druck, Spannung oder Strom konfiguriert oder ausgestaltet ist.

10. Einrichtung nach einem der vorangegangenen Ansprüche, wobei die Einrichtung (200) als eine medizinische Einrichtung (200) eine Blutkassette, ein Blutschlauch oder ein Blutfilter ist.

11. Einrichtung nach einem der vorangegangenen Ansprüche, wobei die Einrichtung (200) ein Einwegprodukt oder ein Disposable ist.

12. Verfahren zum Ermitteln oder Ausgeben eines Werts (Q_abs_korr) einer Eigenschaft (Q) eines in einer Einrichtung, insbesondere einer medizinischen Einrichtung (200), vorliegenden Fluids oder zum Korrigieren eines entsprechenden Signalwertes (U_meas), mit den Schritten:

- Bereitstellen einer Einrichtung (200) gemäß wenigstens einem der vorangegangenen Ansprüche;
- Ausgeben eines Signalwerts (U_meas) mittels eines Wandlers (205, 207, 209, 211) der Einrichtung (200) in Relation zu der Eigenschaft (Q) des Fluids, während dieses in einer der Fluidbahnen (202) vorliegt;

- Bereitstellen des Ergebnisses einer Referenzmessung für die Eigenschaft (dx, dy) als einen ersten Wert (dx_0, dy_0);
- Empfangen eines Signals von der Einrichtung zum Ermitteln einer Eigenschaft (dx, dy) der Einrichtung (200) die Eigenschaft (dx, dy) betreffend als einen zweiten Wert (dx_meas, dy_meas);
- Ausgeben eines Werts (Q_abs_korr) der Eigenschaft (Q) des Fluids, unter Verwenden einer Relation zwischen dem ersten Wert (dx_0, dy_0) und dem zweiten Wert (dx_meas, dy_meas); und/oder Korrigieren des Signalwerts (U_meas) unter Verwenden der Relation zwischen dem ersten Wert (dx_0, dy_0) und dem zweiten Wert (dx_meas, dy_meas).

13. Verfahren nach Anspruch 12, wobei der Schritt des Ausgebens oder des Korrigierens nur erfolgt, wenn eine Differenz zwischen dem ersten Wert (dx_0, dy_0) und dem zweiten Wert (dx_meas, dy_meas) wenigstens einen vorbestimmten Differenzwert erreicht.

14. Behandlungsvorrichtung (100) mit wenigstens je

- einer Einrichtung zum Aufnehmen oder Ankoppeln einer Einrichtung (200) gemäß einem der Ansprüche 1 bis 11 mit einem Wandler (205, 207, 209, 211) an die Behandlungsvorrichtung (100);
- einer Einrichtung zum Bereitstellen des Ergebnisses einer Referenzmessung für die Eigenschaft (dx, dy) als einen ersten Wert (dx_0, dy_0);
- einer Einrichtung zum Empfangen eines Signals von der Einrichtung zum Ermitteln einer Eigenschaft (dx, dy) der Einrichtung (200) die Eigenschaft (dx, dy) betreffend als einen zweiten Wert (dx_meas, dy_meas);
- einer Einrichtung zum Ausgeben eines Werts (Q_abs_korr) der Eigenschaft (Q) des Fluids, unter Verwenden einer Relation zwischen dem ersten Wert (dx_0, dy_0) und dem zweiten Wert (dx_meas, dy_meas); und/oder zum Korrigieren des Signalwerts (U_meas) unter Verwenden der Relation zwischen dem ersten Wert (dx_0, dy_0) und dem zweiten Wert (dx_meas, dy_meas).

15. Set mit einer Behandlungsvorrichtung (100) gemäß Anspruch 14, mit

- einer Einrichtung (200) gemäß einem der Ansprüche 1 bis 11, aufgenommen an der oder angekoppelt an die Behandlungsvorrichtung (100), und mit
- einer Auswerteeinheit (400), programmiert oder konfiguriert zum Ausführen eines Verfahrens mit den Schritten:

- Ausgeben eines Signalwerts (U_meas) mittels eines Wandlers (205, 207, 209, 211) der Einrichtung (200) in Relation zu der Eigenschaft (Q) des Fluids, während dieses in einer der Fluidbahnen (202) vorliegt;
- Bereitstellen des Ergebnisses einer Referenzmessung für die Eigenschaft (dx, dy) als einen ersten Wert (dx_0, dy_0);
- Empfangen eines Signals von der Einrichtung zum Ermitteln einer Eigenschaft (dx, dy) der Einrichtung (200) die Eigenschaft (dx, dy) betreffend als einen zweiten Wert (dx_meas, dy_meas);
- Ausgeben eines Werts (Q_abs_korr) der Eigenschaft (Q) des Fluids, unter Verwenden einer Relation zwischen dem ersten Wert (dx_0, dy_0) und dem zweiten Wert (dx_meas, dy_meas); und/oder Korrigieren des Signalwerts (U_meas) unter Verwenden der Relation zwischen dem ersten Wert (dx_0, dy_0) und dem zweiten Wert (dx_meas, dy_meas).

**Claims**

1. A device (200), in particular a medical device (200), to its reception in or coupling with a treatment apparatus (100), wherein the device (200) is designed with at least, respectively:

   - one hard part (201) with fluid paths (202) for guiding a fluid, in particular a medical fluid, in particular blood, through the hard part (201);
   - one converter (205, 207, 209, 211), wherein the converter (205, 207, 209, 211) is arranged to measure a characteristic (Q) of the fluid while it is present in one of the fluid paths (202); and
   - one device for determining a characteristic, in particular a geometrical characteristic (dx, dy) of the device (200), in particular of a fluid path (202) thereof.

2. The device according to claim 1, wherein the device for determining a characteristic (dx, dy) of the device (200) is or comprises a device for acting on the fluid, in particular for stimulating it to vibrate while the fluid is present in one of the fluid paths (202).

3. The device according to anyone of the preceding claims, wherein the device for determining a characteristic (dx, dy) of the device (200) is or comprises at least one piezo element (225, 227).

4. The device according to claim 3, wherein the piezo element (225, 227) is or comprises one plastic piezo element.

5. The device according to anyone of the preceding claims, wherein at least one section of the converter (205, 207, 209, 211) and/or at least one section of the device for determining a characteristic (dx, dy) of the device (200) is/are or has/have been applied by means of at least one additive application method.

6. The device according to claim 5, wherein the application method is or comprises a template-free application.

7. The device according to claim 5 or 6, wherein the application method comprises application of a conductive ink.

8. The device according to anyone of the preceding claims, wherein at least one converter (205, 207, 209, 211) is an MID flow sensor.

9. The device according to anyone of the preceding claims, wherein the at least one converter (205, 207, 209, 211) is configured or designed for measuring or determining conductivity, flow, pressure, tension or current.

10. The device according to anyone of the preceding claims, wherein the device (200) is a medical device (200), a blood cassette, a blood tube or a blood filter.

11. The device according to anyone of the preceding claims, wherein the device (200) is a single use product or a disposable.

12. A method for determining or outputting a value (Q_abs_korr) of a characteristic (Q) of a fluid present in a device (200), in particular a medical device (200), or for correcting a corresponding signal value (U_meas), comprising the steps of:

    - providing a device (200) according to at least anyone of the preceding claims;
    - outputting a signal value (U_meas) by means of a converter (205, 207, 209, 211) of the device (200) in relation to the characteristic (Q) of the fluid while it is present in one of the fluid paths (202);
    - providing the result of a reference measurement for the characteristic (dx, dy) as a first value (dx_0, dy_0);
    - receiving a signal from the device for determining a characteristic (dx, dy) of the device (200) relating to the characteristic (dx, dy) as a second value (dx_meas, dy_meas);
    - outputting a value (Q_abs_korr) of the characteristic (Q) of the fluid using a relation between the first value (dx_0, dy_0) and the second value (dx_meas, dy_meas); and/or correcting the signal value (U_meas) using the relation between the first value (dy_0, dy_0) and the second value (dx_meas, dy_meas).

13. The method according claim 12, wherein the step of outputting or correcting only occurs if a difference between the first value (dx_0, dy_0) and the second value (dx_meas, dy_meas) reaches at least a predetermined difference value.

14. A treatment apparatus (100) comprising at least, respectively:

> - a device for receiving or coupling a device (200) with a converter (205, 207, 208, 211), according to anyone of claims 1 to 11, to the treatment apparatus (100);
> - a device for providing the result of a reference measurement for the characteristic (dx, dy) as a first value (dx_0, dy_0);
> - a device for receiving a signal from the device for determining a characteristic (dx, dy) of the device (200) relating to the characteristic (dx, dy) as a second value (dx_meas, dy_meas);
> - a device for outputting a value (Q_abs_korr) of the characteristic (Q) of the fluid using a relation between the first value (dx_0, dy_0) and the second value (dx_meas, dy_meas); and/or for correcting the signal value (U_meas) using the relation between the first value (dy_0, dy_0) and the second value (dx_meas, dy_meas).

15. A set with a treatment apparatus (100) according to claim 14, comprising:

> - a device (200), according to anyone of claims 1 to 11, received in or coupled with the treatment apparatus (100), and comprising
> - an evaluation unit (400), programmed or configured to perform a method having the following steps:
>
> > - outputting a signal value (U_meas) by means of a converter (205, 207, 209, 211) of the device (200) in relation to the characteristic (Q) of the fluid while it is present in one of the fluid paths (202);
> > - providing the result of a reference measurement for the characteristic (dx, dy) as a first value (dx_0, dy_0);
> > - receiving a signal from the device for determining a characteristic (dx, dy) of the device (200) relating to the characteristic (dx, dy) as a second value (dx_meas, dy_meas);
> > - outputting a value (Q_abs_korr) of the characteristic (Q) of the fluid using a relation between the first value (dx_0, dy_0) and the second value (dx_meas, dy_meas); and/or correcting the signal value (U_meas) using the relation between the first value (dy_0, dy_0) and the second value (dx_meas, dy_meas).

**Revendications**

1. Un dispositif (200), notamment un dispositif médical, destiné à être reçu ou couplé à un appareil de traitement (100), le dispositif (200) étant conçu avec au moins, respectivement:

> - une partie rigide (201) avec des passages de fluide (202) permettant de guider un fluide, notamment un fluide médical, notamment du sang, au travers de la partie rigide (201);
> - un transducteur (205, 207, 209, 211), où le transducteur (205, 207, 209, 211) est agencé de façon à mesurer une caractéristique (Q) du fluide alors qu'il est présent dans l'un des passages de fluide (202); et
> - un dispositif destiné à déterminer une caractéristique, notamment une caractéristique géométrique (dx, dy) du dispositif (200), notamment d'un passage de fluide (202) de celui-ci.

2. Le dispositif selon la première revendication, où le dispositif destiné à déterminer une caractéristique (dx, dy) du dispositif (200) est, ou comprend, un dispositif destiné à agir sur le fluide, notamment pour le stimuler à vibrer, lorsque que le fluide est présent dans l'un des passages de fluide (202).

3. Le dispositif selon l'une quelconque des revendications précédentes, où le dispositif destiné à déterminer une caractéristique (dx, dy) du dispositif (200) est, ou comprend, au moins un élément piézo (225, 227).

4. Le dispositif selon la revendication 3, où l'élément piézo (225, 227) est, ou comprend, un élément piézo en plastique.

5. Le dispositif selon l'une quelconque des revendications précédentes, où au moins une section du transducteur (205, 207, 209, 211) et/ou au moins une section du dispositif destiné à déterminer une caractéristique (dx, dy) du dispositif (200) est/sont ou a/ont été appliquée (s) au moyen d'au moins un procédé d'application additive.

6. Le dispositif selon la revendication 5, où le procédé d'application est, ou comprend, une application sans masquage.

**7.** Le dispositif selon la revendication 5 ou 6, où le procédé d'application comprend une application d'encre conductrice.

**8.** Le dispositif selon l'une quelconque des revendications précédentes, où au moins un transducteur (205, 207, 209, 211) est un capteur de débit MID.

**9.** Le dispositif selon l'une quelconque des revendications précédentes, où au moins un transducteur (205, 207, 209, 211) est configuré ou conçu pour mesurer ou déterminer la conductivité, le flux, la pression, la tension ou le courant.

**10.** Le dispositif selon l'une quelconque des revendications précédentes, où le dispositif (200) est un dispositif médical (200), une cassette à sang, un tube sanguin ou un filtre sanguin.

**11.** Le dispositif selon l'une quelconque des revendications précédentes, où le dispositif (200) est un produit à usage unique ou jetable.

**12.** Un procédé permettant de déterminer ou de produire la sortie d'une valeur (Q_abs_korr) d'une caractéristique (Q) d'un fluide présent dans un dispositif, notamment un dispositif médical (200) ou de corriger une valeur de signal correspondante (U_meas), comprenant les étapes suivantes:

- la mise à disposition d'un dispositif (200) selon au moins l'une quelconque des revendications précédentes;
- la sortie d'une valeur de signal (U_meas) au moyen d'un transducteur (205, 207, 209, 211) du dispositif (200) en relation avec la caractéristique (Q) du fluide lorsqu'il est présent dans l'un des passages de fluide (202);
- la mise à disposition du résultat d'une mesure de référence pour la caractéristique (dx, dy) en tant que première valeur (dx_0, dy_0);
- la réception d'un signal du dispositif afin de déterminer une caractéristique (dx, dy) du dispositif (200) relative à la caractéristique (dx, dy) en tant que seconde valeur (dx_meas, dy_meas);
- la sortie d'une valeur (Q_abs_korr) de la caractéristique (Q) du fluide en utilisant une relation entre la première valeur (dx_0, dy_0) et la seconde valeur (dx_meas, dy_meas); et/ou la correction de la valeur du signal (U_meas) en utilisant la relation entre la première valeur (dy_0, dy_0) et la seconde valeur (dx_meas, dy_meas).

**13.** Le procédé selon la revendication 12, où l'étape de sortie ou de correction n'a lieu qu'à la condition où une différence entre la première valeur (dx_0, dy_0) et la seconde valeur (dx_meas, dy_meas) atteint au moins une valeur de différence prédéterminée.

**14.** Un appareil de traitement (100) comprenant au moins, respectivement:

- un dispositif destiné à recevoir ou à coupler un dispositif (200) ayant un transducteur (205, 207, 208, 211), selon l'une quelconque des revendications 1 à 11, avec l'appareil de traitement (100);
- un dispositif destiné à fournir le résultat d'une mesure de référence pour la caractéristique (dx, dy) en tant que première valeur (dx_0, dy_0);
- un dispositif destiné à recevoir un signal du dispositif afin de déterminer une caractéristique (dx, dy) du dispositif (200) relative à la caractéristique (dx, dy) en tant que seconde valeur (dx_meas, dy_meas);
- un dispositif destiné à sortir une valeur (Q_abs_korr) de la caractéristique (Q) du fluide en utilisant une relation entre la première valeur (dx_0, dy_0) et la seconde valeur (dx_meas, dy_meas); et/ou à corriger la valeur du signal (U_meas) en utilisant la relation entre la première valeur (dy_0, dy_0) et la seconde valeur (dx_meas, dy_meas).

**15.** Un set avec un appareil de traitement (100) selon la revendication 14, comprenant:

- un dispositif (200), selon l'une quelconque des revendications 1 à 11, reçu ou couplé à l'appareil de traitement (100), et comprenant:
- une unité d'évaluation (400), programmée ou configurée pour exécuter un procédé comportant les étapes suivantes:

- la sortie d'une valeur de signal (U_meas) au moyen d'un transducteur (205, 207, 209, 211) du dispositif (200) en relation avec la caractéristique (Q) du fluide lorsqu'il est présent dans l'un des passages de fluide (202);
- la mise à disposition du résultat d'une mesure de référence pour la caractéristique (dx, dy) en tant que première valeur (dx_0, dy_0);
- la réception d'un signal du dispositif afin de déterminer une caractéristique (dx, dy) du dispositif (200) relative à la caractéristique (dx, dy) en tant que seconde valeur (dx_meas, dy_meas);
- la sortie d'une valeur (Q_abs_korr) de la caractéristi -que (Q) du fluide en utilisant une relation entre la première valeur (dx_0, dy_0) et la seconde valeur (dx_meas, dy_meas); et/ou la correction de la valeur du signal (U_meas) en utilisant la relation entre la première valeur (dy_0, dy_0) et la seconde valeur (dx_meas, dy_meas).

Fig. 1

Fig. 2

Fig. 3

200

201

225

dy

207

202

207

227

dx

Fig. 4

$$\frac{dx\_0}{dy\_0} \longrightarrow \boxed{401} \qquad \boxed{403} \xleftarrow{\frac{dx\_meas}{dy\_meas}} \boxed{225, 227}$$

dx_0     dx_meas
dy_0     dy_meas

$$\boxed{205} \xrightarrow{\begin{array}{c} Q\_abs \\ U\_meas \end{array}} \boxed{405}$$

Q_abs_korr | U_meas_korr

$$\boxed{101}$$

400

Fig. 5

**IN DER BESCHREIBUNG AUFGEFÜHRTE DOKUMENTE**

**In der Beschreibung aufgeführte Patentdokumente**

- DE 102009018664 A1
- US 20030042181 A1
- US 20140263064 A1
- US 20110214504 A1
- EP 2559656 A1